# EUROPEAN PATENT SPECIFICATION

(11) **EP 3 138 527 B1**
(45) Date of publication and mention of the grant of the patent: **20.02.2019**
(21) Application number: 14890543.3
(22) Date of filing: 01.08.2014
(51) Int. Cl.: A61B 90/00, A61G 12/00

(54) **MEDICAL PENDANT BOX BODY**
HÄNGENDER MEDIZINISCHER KASTENKÖRPER
BOÎTIER ALLONGÉ À USAGE MÉDICAL

(30) Priority: 30.04.2014 CN 201410181214
(43) Date of publication of application: 08.03.2017
(73) Proprietor: Maquet (Suzhou) Co. Ltd., Suzhou, Jiangsu 215024 (CN)
(72) Inventor: HUANG, Jiasheng, Suzhou Jiangsu 215024 (CN); ZHANG, Wei, Suzhou Jiangsu 215024 (CN); LI, Qunhua, Suzhou Jiangsu 215024 (CN); JI, Ming, Suzhou Jiangsu 215024 (CN)
(74) Representative: WSL Patentanwälte Partnerschaft mbB
(86) International application number: PCT/CN2014/083500
(87) International publication number: WO 2015/165159

(56) References cited:
- CN-A- 101 940 499
- CN-A- 103 908 350
- CN-U- 201 710 467
- CN-U- 203 815 592
- CN-Y- 201 342 036
- CN-Y- 201 358 676
- US-A- 4 475 322
- US-A- 5 805 075
- US-A- 5 805 075
- US-A- 5 878 536
- US-A1- 2004 020 675
- US-A1- 2004 231 248
- US-B1- 6 668 493
- US-B1- 7 845 601

## Description

### Technical field

The present invention relates to the technical field of medical equipment, and more particularly to a medical pendant box body.

### Background art

Medical pendant system is an indispensable medical equipment used in modern hospital operating room, intensive care unit and the like. The main structure of a medical pendant is a box body. The existing medical pendant box body is generally composed of panels and posts. The installation mode for the panels and posts of existing medical pendant box body lies in that holes are formed in the two sides of the front of the panels, the panels are fixed to the posts using large number of installation screws in a fixed interval, and silica gel or rubber sealing strips are needed to mask the screws after fixing with the screws.

Therefore, the existing medical pendant box body has the following drawbacks:
(1) A large number of installation screws are needed to use, the installation is laborious and time-consuming, and the screws are exposed on the surface of the panels;
(2) silica gel or rubber sealing strips are needed to mask the screws after fixing with the screws if it is not desired for the screws to be exposed on the surface of the panels, but the use of silica gel or rubber sealing strips is not good for the cleaning and disinfection in hospitals.

US 6,668,493 B1 relates to a modular medical gas services unit with multiple medical gas outlets supported at the same level on the column. The unit comprises a hollow column with an internal space for housing the gas conduits and power lines. The medical gas supply outlets are mounted so that their longitudinal axes extend radially from the vertical axis of the frame, and the longitudinal axes of adjacent outlets intersect to form an acute angle. In this way, the horizontal dimensions of the column can be minimized while the number of medical gas outlets at the desired height is maximized. In one embodiment, the column is pentagonal in cross-section providing five planar support surfaces for five medical gas outlets. In another embodiment, a square column is equipped with angled outlet panels, each supporting two medical gas outlets. Thus, though the frame is four-sided, as many as eight medical gas outlets can be mounted at the same height on the frame.

### Summary of invention

One of the purposes of the present invention is to overcome the above-mentioned disadvantages of the existing medical pendant box body, there is provided a medical pendant box body, which can be installed using a small amount of screws or even not using a screw to achieve the reliable connection of the panels and posts as well as the entire box body, without exposed screws on the surface of the panels, without using the silica gel or rubber sealing strips to mask the screws, which facilitates the cleaning and disinfection, being capable of realizing the rapid disassembly and assembly of the medical pendant box body with very short time consumption.

The above purposes of the present invention are achieved by a medical pendant box body, the medical pendant box body comprises at least two panels, at least two posts extending along the longitudinal direction of the medical pendant box body, an upper base plate and a lower base plate, the at least two panels are fixed to the at least two posts, respectively, the upper base plate and the lower base plate are fixed to the upper edge and the lower edge of each panel of the at least two panels, respectively, a snap-fit portion is provided on each panel of the at least two panels at or in the vicinity of its at least one side edge, and a slot for receiving the corresponding snap-fit portion of the corresponding panel is provided on at least one side of each post of the at least two posts, wherein the at least two panels are eight panels, the at least two posts are four posts, the medical pendant box body further comprises four auxiliary columns extending along the longitudinal direction of the medical pendant box body, the eight panels comprises two side panels having generally L shaped cross section and six panels having generally I-shaped cross section, in which the medical pendant box body comprises two sub-box bodies, each sub-box body comprises one side panel having generally L shaped cross section, three panels having generally I-shaped cross section, two posts and two auxiliary columns.

According to the above-mentioned technical solution, the medical pendant box body of the present invention can have the following beneficial technical effects: it can be installed using a small amount of screws or even not using a screw to achieve the reliable connection of the panels and posts as well as the entire box body, without exposed screws on the surface of the panels, without using the silica gel or rubber sealing strips to mask the screws, which facilitates the cleaning and disinfection, being capable of realizing the rapid disassembly and assembly of the medical pendant box body with very short time consumption.

Preferably, the upper base plate and the lower base plate each comprises a through-hole at or the in the vicinity of its periphery for a base plate-fixing screw to pass through, and a base plate-fixing threaded hole for receiving the corresponding base plate-fixing screw is provided at or in the vicinity of the upper edge and lower edge of each panel of the at least two panels.

According to the above-mentioned technical solution, the medical pendant box body of the present invention can have the following beneficial technical effects: the number of the screws used in the medical pendant box body of the present invention is greatly reduced as compared with the number of the screws used in the medical pendant box body of the prior art, furthermore, there is no exposed screws on the surface of the panels, without using the silica gel or rubber sealing strips to mask the screws, which facilitates the cleaning and disinfection, being capable of realizing the rapid disassembly and assembly of the medical pendant box body with very short time consumption.

Since the at least two panels are eight panels, the at least two posts are four posts, and the medical pendant box body further comprises four auxiliary columns extending along the longitudinal direction of the medical pendant box body, wherein the eight panels comprises two side panels having generally L shaped cross section and six panels having generally I-shaped cross section, in which the medical pendant box body comprises two sub-box bodies, and each sub-box body comprises one side panel having generally L shaped cross section, while three panels having generally I-shaped cross section, two posts and two auxiliary columns the medical pendant box body of the present invention can have the following beneficial technical effects: a medical pendant box body having relatively large space of the box body and rational space distribution is provided, which can be installed using a small amount of screws or even not using a screw to achieve the reliable connection of the panels and posts as well as the entire box body, without exposed screws on the surface of the panels, being capable of realizing the rapid disassembly and assembly of the medical pendant box body.

Preferably, the total cross section of the medical pendant box body is generally rectangular or trapezoidal, and the cross section of each sub-box body is generally square or trapezoidal.

According to the above-mentioned technical solution, the medical pendant box body of the present invention can have the following beneficial technical effects: a medical pendant box body and its sub-box body having preferable cross section shape are provided, which can realize the simple and rapid disassembly and assembly of the medical pendant box body using less screws, meanwhile can more appropriately accommodate the internal components of the box body.

Preferably, there is an interval between the two sub-box bodies, which forms a hollow area from top to bottom.

According to the technical solutions described above, the medical pendant box body of the present invention can have the following beneficial technical effects: the hollow area can be used as the cable management area of the medical pendant.

Preferably, the auxiliary columns are provided with panel-fixing thread grooves for receiving the panel-fixing screws so as to fix at least one panel to the auxiliary columns.

According to the technical solutions described above, the medical pendant box body of the present invention can have the following beneficial technical effect: the firmness of the medical pendant box body is further reinforced through the additional fixation of the auxiliary columns and the panel-fixing screws.

Preferably, the medical pendant box body also comprises a side cover plate for masking the panel-fixing screws.

According to the above-mentioned technical solution, the medical pendant box body of the present invention can have the following beneficial technical effects: in the case of setting the auxiliary columns and panel-fixing screws, the panel-fixing screws are effectively masked, so that the screws are not exposed on the surface of the panels, without using the silica gel or rubber sealing strips to mask the screws, which facilitates the cleaning and disinfection.

Preferably, at least two spare thread grooves are provided on the internal surface of each panel of the at least two panels.

According to the above-mentioned technical solution, the medical pendant box body of the present invention can have the following beneficial technical effects: the installation of the air port of the panel can be implemented without exposing screws, which changes the phenomenon that a large number of screws are exposed after installing the air port in the panels of the box body of the prior art, which facilitates the cleaning and disinfection in hospitals.

Preferably, the distances among the at least two spare thread grooves on each panel are equal.

According to the above-mentioned technical solution, the medical pendant box body of the present invention can have the following beneficial technical effects: when electrical isolation is needed among the panels, electrical separators can be made into a general part owing to the uniform interval among the spare thread grooves, so that the electrical separators of different panels can totally be installed interchangeably, which strengthens the versatility of the electrical separators.

Preferably, the medical pendant box body also comprises a separator, the edge part of the separator pressing against the top surface of the periphery of the groove of the spare thread grooves via the head of the separator-fixing screw.

According to the technical solutions described above, the medical pendant box body of the present invention can have the following beneficial technical effect: the separator can be rapidly and simply installed on the panel without exposing the separator-fixing screw.

Preferably, the cross section of the separator is generally in a shape which looks like the Chinese character " 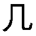 (ji)".

According to the above-mentioned technical solution, the medical pendant box body of the present invention can have the following beneficial technical effects: the electrical isolation can be achieved, meanwhile can more appropriately accommodate the internal components of the box body.

Preferably, the medical pendant box body also comprises auxiliary columns extending along the longitudinal direction of the medical pendant box body, the auxiliary columns are provided with panel-fixing thread grooves for receiving the panel-fixing screws so as to fix at least one panel to the auxiliary columns.

According to the technical solutions described above, the medical pendant box body of the present invention can have the following beneficial technical effect: the firmness of the medical pendant box body is further reinforced through the additional fixation of the auxiliary columns and the panel-fixing screws.

### Brief description of drawings

Figure 1 is the three-dimensional view for the medical pendant box body of the first embodiment not belonging to the claimed invention.
Figure 2 is the three-dimensional view for the medical pendant box body of the first embodiment, in which the upper base plate is removed for clarity.
Figure 3 is the top view of the medical pendant box body shown in figure 2.
Figure 4 is the partial enlarged view of a portion of the top left corner of figure 3, in which the portion of the top left corner of figure 3 is rotated by 180 degrees for clarity.
Figure 5 is the three-dimensional view for the medical pendant box body of the second embodiment, which belongs to the present invention.
Figure 6 is the three-dimensional view for the medical pendant box body of the second embodiment belonging to the present invention, in which the upper base plate is removed for clarity.
Figure 7 is the top view of the medical pendant box body shown in figure 6, in which the anterior-posterior direction is turned by 180 degrees for clarity.
Figure 8 is the partial enlarged view of a portion of the top left corner of figure 7.
Figure 9 is the partial enlarged view showing the details of the spare thread grooves and separator in the medical pendant box body.

### Sign list for drawings

1. panel;
2. post;
3. upper base plate;
4. lower base plate;
5. side cover plate;
6. auxiliary columns;
10. medical pendant box body;
11. snap-fit portion;
12. base plate-fixing threaded holes;
13. spare thread grooves;
14. separator;
15. top surface of periphery of the groove;
21. slot;
22. panel-fixing thread grooves;
31. mechanical connecting piece;
101. sub-box body;
102. sub-box body.

### Detailed description

The present invention is further described in connection with drawings and particular embodiments as follows and elaborated in more detail in the following description in order to fully understand the present invention, but it is evident that the present invention can be implemented in many other ways which are different from those described herein; generalization and deduction can be made by a skilled in the art without departing from the connotation of the invention according to practical application, and therefore the protective scope of the present invention should not be limited by the specific content of embodiments of the present invention herein.

### (The first embodiment)

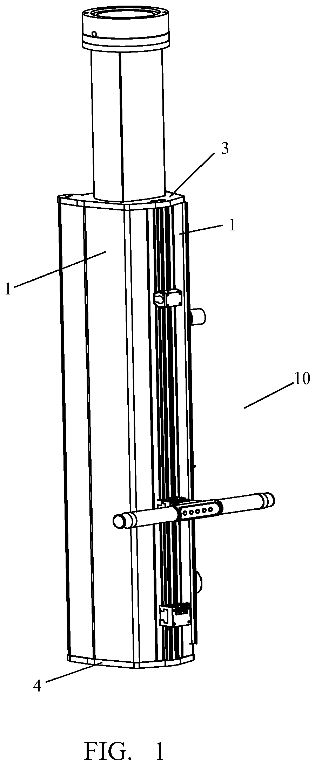

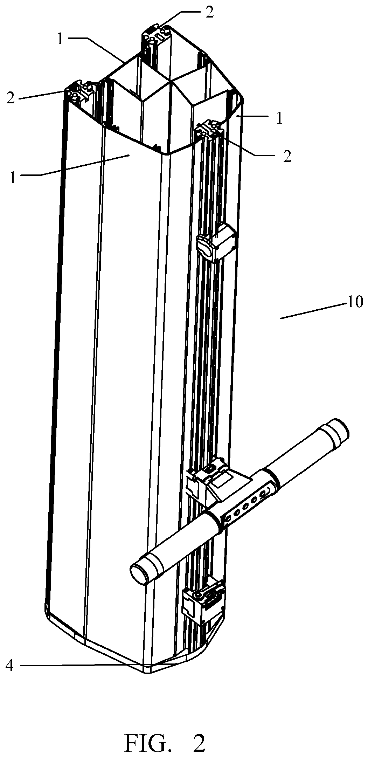

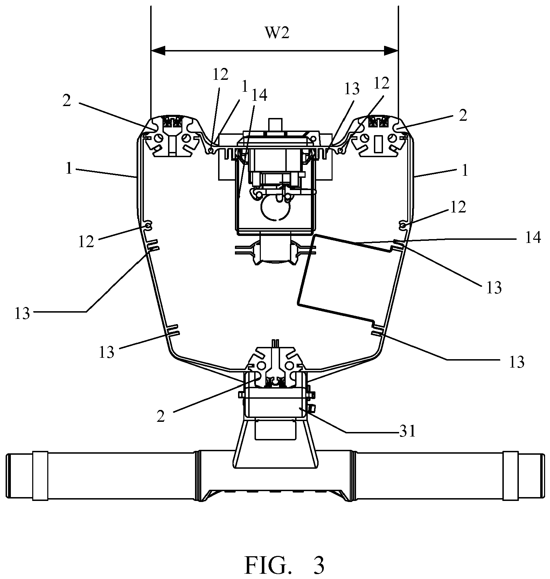

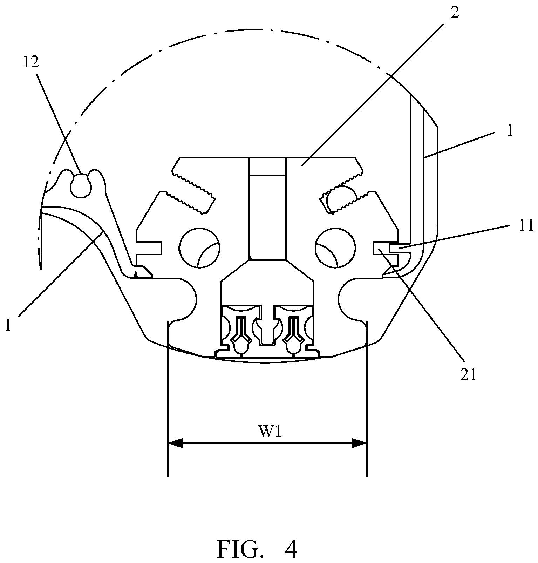

Figure 1 and figure 2 respectively show the three-dimensional views for the medical pendant box body 10 of the first embodiment not belonging to the claimed invention, in which the upper base plate is removed from the medical pendant box body 10 in figure 2 for clarity. Figure 3 shows the top view of the medical pendant box body 10 shown in figure 2. Figure 4 shows the partial enlarged view of a portion of the top left corner of figure 3, in which the portion of the top left corner of figure 3 is rotated by 180 degrees for clarity.

The medical pendant box body 10 comprises at least two panels 1, at least two posts 2 extending along the longitudinal direction of the medical pendant box body, an upper base plate 3 and a lower base plate 4, at least two panels 1 are fixed to the at least two posts 2, respectively, the upper base plate 3 and the lower base plate 4 are fixed to the upper edge and the lower edge of each panel 1 of at least two panels 1, respectively, a snap-fit portion 11 is provided on or near to at least one side edge of each panel 1 of at least two panels 1, and a slot 21 is provided on at least one side of each post 2 of at least two posts 2 for receiving the corresponding snap-fit portion 11 of the corresponding panel 1.

In this way, the medical pendant box body can be installed using a small amount of screws or even not using a screw to achieve the reliable connection of the panels and posts as well as the entire box body, without exposed screws on the surface of the panels, without using the silica gel or rubber sealing strips to mask the screws, which facilitates the cleaning and disinfection, being capable of realizing the rapid disassembly and assembly of the medical pendant box body with very short time consumption.

Note that, "upper", "lower", "front", "rear", "left", "right" and the like used herein are only exemplary directions defined to facilitate the description of the invention, as shown in figure 3, the direction toward the reader is "upper", the direction away from the reader is "lower", the direction of the bottom side in the paper is "front", the direction of the top side in the paper is "rear", the direction of the left side in the paper is "left", and the direction of the right side in the paper is "right". Of course, those skilled in the art on the basis of the present invention can understood that "upper", "lower", "front", "rear", "left", "right" and other directions can also be defined in other ways, which also fall into the protective scope of the present invention.

Preferably, the upper base plate 3 and the lower base plate 4 each comprises a through-hole (not shown) at or the in the vicinity of its periphery for a base plate-fixing screw (not shown) to pass through, and a base plate-fixing threaded hole 12 is provided at or in the vicinity of the upper edge and lower edge of each panel 1 of at least two panels 1 for receiving the corresponding base plate-fixing screw.

Although the embodiments given above only describe the case of the additional fixation of the base plates and the panels through the base plate-fixing screws, a person skilled in the art on the basis of the present invention will understand that other fixing modes (for example, riveting, welding, etc.) can also be employed as long as the base plates can be fixed to the panels. Such variation also falls into the protection scope of the present invention.

In this way, even though the base plates and the panels are additionally fixed via the base plate-fixing screws, the number of the screws (for example, in the embodiment shown in figure 3, only need totally upper and lower eight screws) used in the medical pendant box body of the present invention has been also greatly reduced as compared with the number of the screws (generally need several dozens) used in the medical pendant box body of the prior art, furthermore, there is no exposed screws on the surface of the panels (for example, in the embodiment shown in figure 3, the screws are located at the upper base plate and the lower base plate), without using the silica gel or rubber sealing strips to mask the screws, which facilitates the cleaning and disinfection, being capable of realizing the rapid disassembly and assembly of the medical pendant box body with very short time consumption.

According to the first embodiment not belonging to the claimed invention, preferably, as shown in figures 1-4, the medical pendant box body 10 comprises three panels 1 and three posts 2, the three panels 1 comprises two side panels with generally L-shaped cross section and one rear panel with generally I-shaped cross section.

According to the first embodiment, preferably, as shown in figures 1-4, the cross section of the medical pendant box body 10 is generally square or trapezoidal.

As shown in figures 3 and 4, the medical pendant box body of the first embodiment of the present invention can be adapted for the mechanical connecting piece 31 with width W1 or W2. That is, a single post can be adapted for a mechanical connecting piece with a width of W1, and the interval between two posts can be adapted for a mechanical connecting piece with a width of W2. Preferably, W2 > W1. The mechanical connecting piece can be used for installing various medical accessories, for example, a control handle, an infusion pole, a display arm, a medical guide rail, etc.

### (The second embodiment)

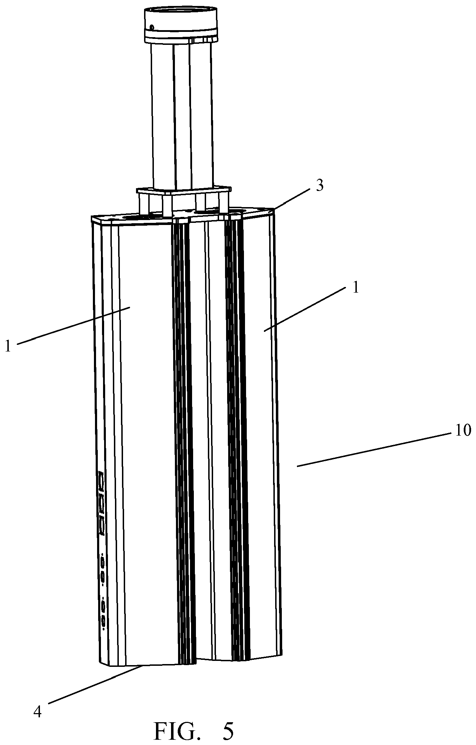

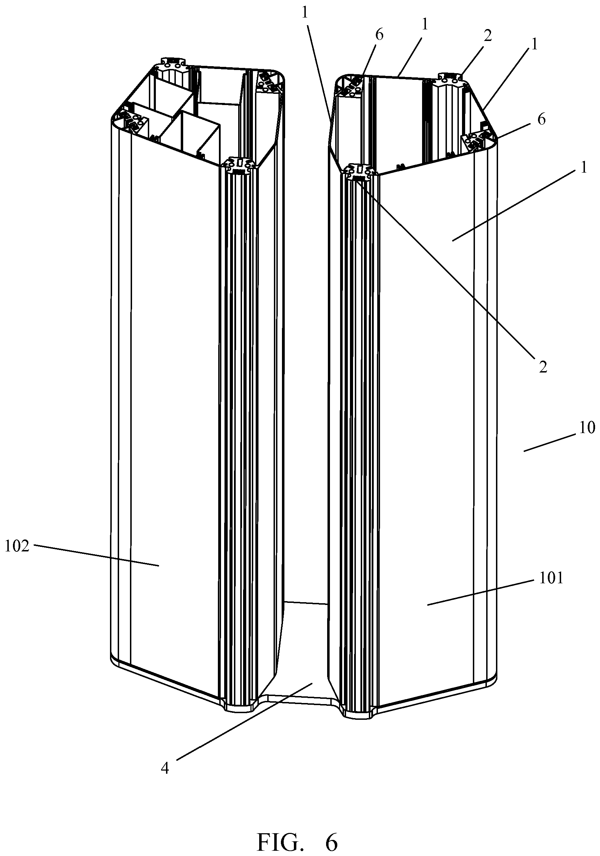

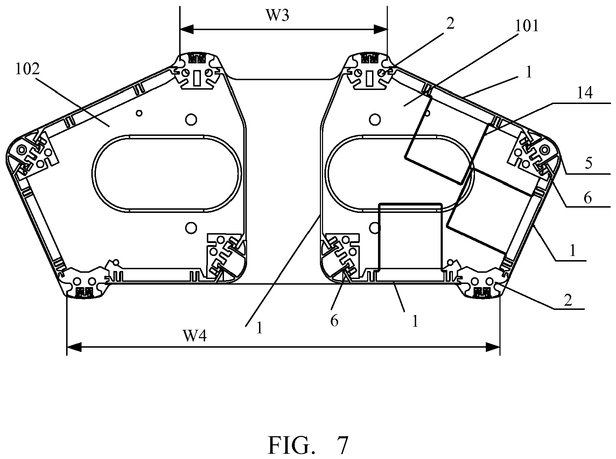

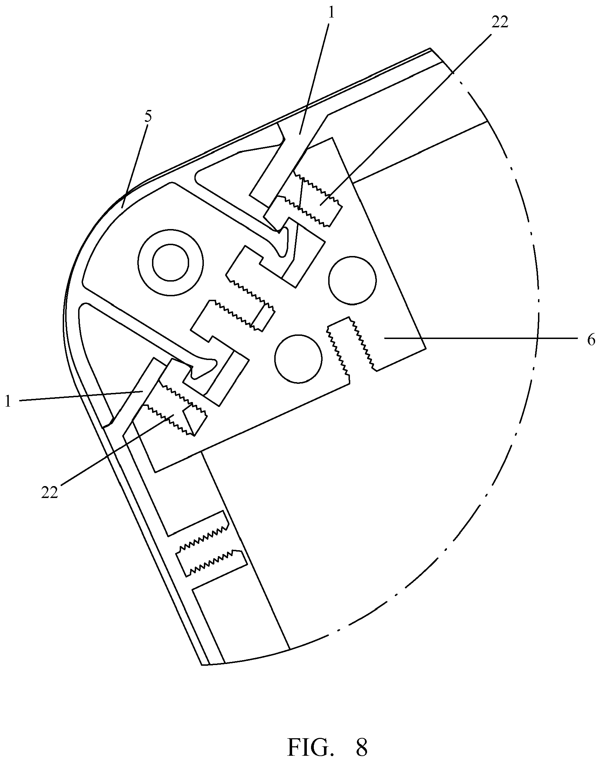

Figure 5 and figure 6 respectively show the three-dimensional views for the medical pendant box body 10 of the second embodiment, which belongs to the present invention, in which the upper base plate is removed from the medical pendant box body 10 in figure 6 for clarity. Figure 7 shows the top view of the medical pendant box body 10 shown in figure 6, in which the anterior-posterior direction is turned by 180 degrees for clarity. Figure 8 shows the partial enlarged view of a portion of the top left corner of figure 7.

According to the second embodiment of the present invention, preferably, as shown in figures 5-8, the medical pendant box body 10 comprises eight panels 1, four posts 2 and four auxiliary columns 6 extending along the longitudinal direction of the medical pendant box body, the eight panels 1 comprises two side panels having generally L shaped cross section and six panels having generally I-shaped cross section, in which the medical pendant box body 10 comprises two sub-box bodies 101 and 102, each sub-box body comprises one side panel having generally L shaped cross section, three panels having generally I-shaped cross section, two posts 2 and two auxiliary columns 6 (in figures 6 and 7, only the panels, posts, and auxiliary columns of the box body 101 are marked for clarity, but it should be understood that the constitution of the sub-box body 102 can be similar to the constitution of the sub-box body 101).

According to the second embodiment of the present invention, preferably, as shown in figures 5-8, the total cross section of the medical pendant box body 10 is generally rectangular or trapezoidal, and the cross section of each sub-box body is generally square or trapezoidal.

According to the second embodiment of the present invention, preferably, as shown in figures 5-8, there is an interval between the two sub-box bodies, which forms a hollow area from top to bottom. In this way, the hollow area can be used as the cable management area of the medical pendant.

As compared with the medical pendant box body of first embodiment of the present invention, the medical pendant box body of the second embodiment of the present invention can provide a larger space of box body and can be adapted for a mechanical connecting piece with wider width. For example, as shown in figure 7, the medical pendant box body of the second embodiment of the present invention can be adapted for the mechanical connecting pieces with widths W3 and W4. That is, the interval between the front two posts can be adapted for a mechanical connecting piece with a width of W3, and the interval between the rear two posts can be adapted for a mechanical connecting piece with a width of W4. Preferably, W4 > W3.

Preferably, the W3 can be made equal to W2 in order to make a mechanical connecting piece of the same width can be used for both the medical pendant box body of the first embodiment of the present invention and used for the second embodiment of the present invention.

As compared with the medical pendant box body of first embodiment of the present invention, the medical pendant box body of the second embodiment of the present invention has larger space of box body and more number of panels and posts, therefore, in order to conduct additional fixation, preferably, as shown in figures 5-8, auxiliary columns 6 are provided with panel-fixing thread grooves 22 (in particular, see figure 8) for receiving panel-fixing screws (not shown) so as to fix at least one panel to the auxiliary columns. The medical pendant box body 10 also comprises a side cover plate 5 for masking the panel-fixing screws.

### (The third embodiment)

The medical pendant box body of the third embodiment not belonging to the claimed invention is substantially the same as the medical pendant box body of the second embodiment of the present invention (therefore, it is not illustrated), the difference therebetween only lies in that: see figure 7, the two side panels with generally L-shaped cross section in the medical pendant box body of the second embodiment of the present invention are removed, and two panels respectively located at the front side and the rear side and having generally I-shaped cross section are added.

That is to say, according to the third embodiment, preferably, the medical pendant box body 10 comprises eight panels 1, four posts 2 and four auxiliary columns 6, the eight panels 1 include eight panels having generally I-shaped cross section.

According to the third embodiment, preferably, the cross section of the medical pendant box body 10 is generally rectangular or trapezoidal.

Although the three embodiments given above only describe the cases of a particular number of panels and posts, and medical pendant box bodies having a particular cross section shape, a person skilled in the art on the basis of the present invention should understand that the protective scope of the present invention is not limited to the particular number of panels and posts, and the medical pendant box bodies having a particular cross section shape listed abovelisted above, and other number of panels and posts and the medical pendant box bodies having other cross section shapes can also be employed as far as encompassed by the claims.

Owing to the numerous variations, other number of panels and posts, and the medical pendant box bodies having other cross section shapes are not listed one by one, all of them fall into the protective scope of the present invention.

Furthermore, a person skilled in the art on the basis of the present invention should understand that auxiliary columns can be set according to needs or no auxiliary column is set, and if auxiliary columns are set, the setting modes of auxiliary columns are not limited to the embodiments give above, and other setting modes can be employed, all of them falling into the protective scope of the present invention. That is to say, in addition to the panels, the posts, the upper base plate and the lower base plate, the medical pendant box body 10 can also comprises auxiliary columns 6 extending along the longitudinal direction of the medical pendant box body, the auxiliary columns 6 are provided with panel-fixing thread grooves 22 for receiving the panel-fixing screws so as to fix at least one panel 1 to auxiliary columns 6.

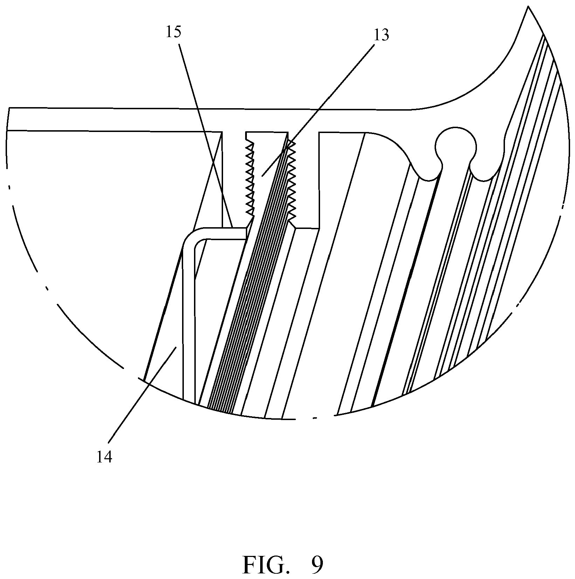

As shown in figures 3 and 9, preferably, at least two spare thread grooves 13 are provided on the internal surface of panels 1. Preferably, these spare thread grooves 13 can extend along the longitudinal direction of the medical pendant box body and passing through the entire length of the medical pendant box body.

Preferably, the distances among the at least two spare thread grooves 13 on each panel 1 are equal. For example, as shown in figure 3, the distance between the two spare thread grooves 13 on the left panel is equal to the distance between the two spare thread grooves 13 on the right panel.

Preferably, the medical pendant box body 10 also comprises a separator 14, the edge part of the separator 14 pressing against the top surface 15 of the periphery of the groove of the spare thread grooves 13 via the head (not shown) of the separator-fixing screw.

Preferably, separator 14 is an electrical separator so as to achieve the electrical isolation among the various internal components in the medical pendant box body 10.

Preferably, the cross section of the separator 14 is generally in a shape which looks like the Chinese character " 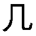 (ji)".

Preferably, as shown in figures 2 and 6, posts 2 extend along the longitudinal direction of the medical pendant box body 10 and passing through the entire length of the medical pendant box body 10. In this way, by means of a mechanical connecting piece, the location of the medical accessories can be steplessly adjusted along the longitudinal direction of the medical pendant box body.

The present invention has been exemplarily described above in connection with the figures, although the specific implementations of the present invention are not limited to the above embodiments. Various modifications or variations can be made by a person skilled in the art on the premise of without departing from the technical concept of the present invention, as defined by the appended claims.

## Claims

1. A medical pendant box body (10), wherein in that the medical pendant box body (10) comprises at least two panels (1), at least two posts (2) extending along the longitudinal direction of the medical pendant box body (10), an upper base plate (3) and a lower base plate (4), the at least two panels (1) are fixed to the at least two posts (2), respectively, the upper base plate (3) and the lower base plate (4) are fixed to the upper edge and the lower edge of each panel of the at least two panels (1), respectively, **characterized in that** a snap-fit portion (11) is provided on or near to at least one side edge of each panel of the at least two panels (1) and a slot (21) for receiving the corresponding snap-fit portion (11) of the corresponding panel (1) is provided on at least one side of each post of the at least two posts (2), wherein the at least two panels (1) are eight panels (1), the at least two posts (2) are four posts (2), the medical pendant box body (10) further comprises four auxiliary columns (6) extending along the longitudinal direction of the medical pendant box body (10), the eight panels (1) comprises two side panels having generally L shaped cross section and six panels having generally I-shaped cross section, in which the medical pendant box body (10) comprises two sub-box bodies (101, 102), each sub-box body (101, 102) comprises one side panel having generally L shaped cross section, three panels (1) having generally I-shaped cross section, two posts (2) and two auxiliary columns (6).

2. The medical pendant box body (10) of claim 1, wherein the upper base plate (3) and the lower base plate (4) each comprises a through-hole at or in the vicinity of its periphery for a base plate-fixing screw to pass through, and a base plate-fixing threaded hole (12) for receiving the corresponding base plate-fixing screw is provided in each panel (1) of the at least two panels at or in the vicinity of its upper edge and lower edge.

3. The medical pendant box body (10) of claim 1, wherein the total cross section of the medical pendant box body (10) is generally rectangular or trapezoidal, and the cross section of each sub-box body (101, 102) is generally square or trapezoidal.

4. The medical pendant box body (10) of claim 1, wherein there is an interval between the two sub-box bodies (101, 102), which forms a hollow area from top to bottom.

5. The medical pendant box body (10) of claim 1, wherein the auxiliary columns (6) are provided with panel-fixing thread grooves (22) for receiving the panel-fixing screws so as to fix at least one panel (1) to the auxiliary columns (6).

6. The medical pendant box body (10) of claim 5, wherein the medical pendant box body (10) also comprises a side cover plate (5) for masking the panel-fixing screws.

7. The medical pendant box body (10) of claim 1, wherein at least two spare thread grooves (13) are provided on the internal surface of each panel (1) of the at least two panels (1).

8. The medical pendant box body (10) of claim 7, wherein the distances among the at least two spare thread grooves (13) on each panel (1) are equal.

9. The medical pendant box body (10) of claim 7, wherein the medical pendant box body (10) also comprises a separator (14), the edge part of the separator (14) pressing against the top surface of the periphery of the groove of the spare thread grooves (13) via the head of the separator-fixing screw.

10. The medical pendant box body (10) of claim 9, wherein the cross section of the separator (14) is generally in a shape which looks like the Chinese character " 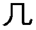 (ji)".

## Patentansprüche

1. Medizinischer Hängekastenkörper (10), wobei der medizinische Hängekastenkörper (10) mindestens zwei Platten (1), mindestens zwei Pfosten (2), welche sich entlang der Längsrichtung des medizinischen Hängekastenkörpers (10) erstrecken, eine obere Grundplatte (3) und eine untere Grundplatte (4) aufweist, wobei die mindestens zwei Platten (1) jeweils an den mindestens zwei Pfosten (2) befestigt sind, wobei die obere Grundplatte (3) und die untere Grundplatte (4) an der oberen Kante und der unteren Kante von jeder der mindestens zwei Platten (1) befestigt sind, **dadurch gekennzeichnet, dass** ein Schnappabschnitt (11) auf oder nahe mindestens einer Seitenkante von jeder der mindestens zwei Platten (1) ausgebildet ist, und eine Aufnahme (21) zur Aufnahme des entsprechenden Schnappabschnitts (11) der entsprechenden Platte (1) an mindestens einer Seite von jedem Pfosten der mindestens zwei Pfosten (2) ausgebildet ist, wobei die mindestens zwei Platten (1) acht Platten (1) sind, wobei die mindestens zwei Pfosten (2) vier Pfosten (2) sind, wobei der medizinische Hängekastenkörper (10) weiter vier Hilfssäulen (6) aufweist, welche sich entlang der Längsrichtung des medizinischen Hängekastenkörpers (10) erstrecken, wobei die acht Platten (1) zwei Seitenplatten mit einem im Wesentlichen L-förmigen Querschnitt und sechs Platten mit einem im Wesentlichen I-förmigen Querschnitt aufweisen, wobei der medizinische Hängekastenkörper (10) zwei Subkastenkörper (101, 102) aufweist, wobei jeder Subkastenkörper (101, 102) eine Seitenplatte mit einem im Wesentlichen L-förmigen Querschnitt und drei Platten (1) mit einem im Wesentlichen I-förmigen Querschnitt, zwei Pfosten (2) und zwei Hilfssäulen (6) aufweist.

2. Medizinischer Hängekastenkörper (10) nach Anspruch 1, wobei die obere Grundplatte (3) und die untere Grundplatte (4) jeweils eine Durchgangsbohrung an ihrem oder in der Nähe ihres Umfangs zum Hindurchführen einer Grundplattenbefestigungsschraube und eine Grundplattenbefestigungsgewindebohrung (12) zur Aufnahme der entsprechenden Grundplattenbefestigungsschraube in jeder Platte (1) der mindestens zwei Platten an oder in der Nähe seiner oberen Kante und seiner unteren Kante aufweisen.

3. Medizinischer Hängekastenkörper (10) nach Anspruch 1, wobei der Gesamtquerschnitt des medizinischen Hängekastenkörpers (10) im Wesentlichen rechteckig oder trapezförmig ist, und der Querschnitt jedes Subkastenkörpers (101, 102) im Wesentlichen quadratisch oder trapezförmig ist.

4. Medizinischer Hängekastenkörper (10) nach Anspruch 1, wobei zwischen den zwei Subkastenkörpern (101, 102) ein Abstand vorhanden ist, welcher von oben nach unten einen Hohlraum bildet.

5. Medizinischer Hängekastenkörper (10) nach Anspruch 1, wobei die Hilfssäulen (6) mit Plattenbefestigungsgewindenuten (22) zur Aufnahme der Plattenbefestigungsschrauben ausgestaltet sind, um mindestens eine Platte (1) an den Hilfssäulen (6) zu befestigen.

6. Medizinischer Hängekastenkörper (10) nach Anspruch 5, wobei der medizinische Hängekastenkörper (10) auch eine Seitenabdeckplatte (5) zum Abdecken der Plattenbefestigungsschrauben aufweist.

7. Medizinischer Hängekastenkörper (10) nach Anspruch 1, wobei mindestens zwei Ersatzgewindenuten (13) auf der inneren Fläche jeder Platte (1) der mindestens zwei Platten (1) ausgebildet sind.

8. Medizinischer Hängekastenkörper (10) nach Anspruch 7, wobei die Abstände zwischen den mindestens zwei Ersatzgewindenuten (13) auf jeder Platte (1) gleich sind.

9. Medizinischer Hängekastenkörper (10) nach Anspruch 7, wobei der medizinische Hängekastenkörper (10) auch eine Trennvorrichtung (14) aufweist, wobei der Kantenteil der Trennvorrichtung (14) über den Kopf der Trennvorrichtungsbefestigungsschraube gegen die obere Fläche des Umfangs der Nut der Ersatzgewindenuten (13) drückt.

10. Medizinischer Hängekastenkörper (10) nach Anspruch 9, wobei der Querschnitt der Trennvorrichtung (14) im Wesentlichen in einer Form ist, welche wie der chinesische Buchstabe " 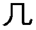 (ji)" aussieht.

## Revendications

1. Corps de boîtier suspendu médical (10), caractérisé dans lequel le corps de boîtier suspendu médical (10) comprend au moins deux panneaux (1), au moins deux montants (2) s'étendant le long de la direction longitudinale du corps de boîtier suspendu médical (10), une plaque de base supérieure (3) et une plaque de base inférieure (4), les au moins deux panneaux (1) sont fixés aux au moins deux montants (2), respectivement, la plaque de base supérieure (3) et la plaque de base inférieure (4) sont fixées au bord supérieur et au bord inférieur de chaque panneau des au moins deux panneaux (1), respectivement, **caractérisé en ce qu'**une partie d'encliquetage (11) est prévue au moins sur ou à proximité d'au moins un bord latéral de chaque panneau des au moins deux panneaux (1) et une fente (21) destinée à recevoir la partie encliquetable (11) correspondante du panneau correspondant (1) est prévue sur au moins un côté de chaque montant des au moins deux montants (2), dans lequel les au moins deux panneaux (1) sont huit panneaux (1), les au moins deux montants (2) sont quatre montants (2), le corps de boîtier suspendu médical (10) comprend en outre quatre colonnes auxiliaires (6) s'étendant dans la direction longitudinale du corps de boîtier suspendu médical (10), les huit panneaux (1) comprennent deux panneaux latéraux ayant une section transversale généralement en forme de L et six panneaux ayant une section transversale généralement en forme de I, où le corps de boîtier suspendu médical (10) comprend deux sous-corps de boîtier (101, 102), chaque sous-corps de boîtier (101, 102) comprend un panneau latéral ayant une section transversale généralement en forme de L, trois panneaux (1) ayant une section transversale généralement en forme de I, deux montants (2) et deux colonnes auxiliaires (6).

2. Corps de boîtier suspendu médical (10) selon la revendication 1, dans lequel la plaque de base supérieure (3) et la plaque de base inférieure (4) comprennent chacune un trou traversant au niveau de ou à proximité de sa périphérie pour le passage traversant d'une vis de fixation de plaque de base, et un trou fileté de fixation de plaque de base (12) destiné à recevoir la vis de fixation de plaque de base correspondante est prévu dans chaque panneau (1) des au moins deux panneaux au niveau ou à proximité de son bord supérieur et de son bord inférieur.

3. Corps de boîtier suspendu médical (10) selon la revendication 1, dans lequel la section transversale totale du corps de boîtier suspendu médical (10) est généralement rectangulaire ou trapézoïdale, et la section transversale de chaque sous-corps de boîtier (101, 102) est généralement carrée ou trapézoïdale.

4. Corps de boîtier suspendu médical (10) selon la revendication 1, dans lequel il existe un intervalle entre les deux sous-corps de boîtier (101, 102), qui forme une zone creuse de haut en bas.

5. Corps de boîtier suspendu médical (10) selon la revendication 1, dans lequel les colonnes auxiliaires (6) sont pourvues de gorges filetées de fixation de panneau (22) pour recevoir les vis de fixation de panneau afin de fixer au moins un panneau (1) aux colonnes auxiliaires (6).

6. Corps de boîtier suspendu médical (10) selon la revendication 5, dans lequel le corps de boîtier suspendu médical (10) comprend également une plaque de recouvrement latérale (5) destinée à masquer les vis de fixation de panneau.

7. Corps de boîtier suspendu médical (10) selon la revendication 1, dans lequel au moins deux gorges filetées supplémentaires (13) sont prévues sur la surface interne de chaque panneau (1) des au moins deux panneaux (1).

8. Corps de boîtier suspendu médical (10) selon la revendication 7, dans lequel les distances entre les au moins deux gorges filetées supplémentaires (13) sur chaque panneau (1) sont égales.

9. Corps de boîtier suspendu médical (10) selon la revendication 7, dans lequel le corps de boîtier suspendu médical (10) comprend également un séparateur (14), la partie de bord du séparateur (14) appuyant contre la surface supérieure de la périphérie de la gorge des gorges filetées supplémentaires (13) via la tête de la vis de fixation de séparateur.

10. Corps de boîtier suspendu médical (10) selon la revendication 9, dans lequel la section transversale du séparateur (14) a généralement une forme qui ressemble au caractère chinois « 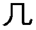 (ji) ».
